# EUROPÄISCHE PATENTANMELDUNG

(11) **EP 1 151 734 A2**
(43) Veröffentlichungstag der Anmeldung: **07.11.2001**
(21) Anmeldenummer: 01115950.6
(22) Anmeldetag: 30.06.1995
(51) Int. Cl.: A61F 13/514, A61F 13/53, A61F 13/15

(54) **Absorbierender Artikel**

(30) Priorität: 30.06.1994 DE 4422956
(62) Teilanmeldung aus: 95925799.9
(71) Anmelder: Kimberly-Clark GmbH, 56070 Koblenz (DE)
(72) Erfinder: Raidel, Maria, Dr., 90451 Nürnberg (DE); Ullmann, Jan, 90402 Nürnberg (DE); Aschenbrenner, Franz, 92280 Kastl (DE)
(74) Vertreter: Engelhard, Elisabeth, Dr.

(57) **Zusammenfassung**

Die Erfindung betrifft einen absorbierenden Artikel mit einer flüssigkeitsundurchlässigen Abdecklage, einer flüssigkeitsdurchlässigen Abdecklage, und einem zwischen flüssigkeitsundurchlässiger Abdecklage und flüssigkeitsdurchlässiger Abdecklage angeordneten Saugkörper wobei die flüssigkeitsundurchlässige Abdecklage und/oder der Saugkörper zumindest in Teilbereichen eine gewellte Form aufweisen und der Saugkörper mindestens eine gewellte Bahn enthält.

Der Saugkörper enthält ein Kardenvlies, ein Spinnvlies, ein Bahnmaterial aus einer Zellstoff-Kunstfasermischung, eine verfestigte luftgelegte Zellstoffbahn, eine luftgelegte Zellstoff-Kunstfasermischung oder Kunstfaservlies.

## Beschreibung

Die Erfindung betrifft einen absorbierenden Artikel mit einer zumindest in Teilbereichen gewellten Bahn aus dünnem, blattförmigem und zumindest in Querrichtung dehnbarem Material.

Aus der deutschen Auslegeschrift DE 2011802 B2 ist eine Vorrichtung bekannt, die sich mit der Wellung einer Papierbahn befaßt. Das Führungsbett der bekannten Vorrichtung muß wegen der Materialeigenschaften des verarbeiteten Papiers einen geometrisch sehr komplizierten, räumlich gekrümmten Verlauf aufweisen, der einen sehr hohen fertigungstechnischen Aufwand erfordert und somit nachteilhaft ist.

Aus der deutschen Patentschrift DE 2945395 C2 ist eine Formplatte für eine Falteinrichtung einer Biesennähmaschine, bestehend aus einer Grundplatte und einer Mehrzahl von Faltrippen an ihrer Oberseite, die parallel verlaufende, über den einen Rand der Grundplatte hinausragende Abschnitte und auf der Grundplatte radial bzw. fächerförmig auseinanderlaufende Abschnitte aufweisen, bekannt. Die Faltrippen sind einzelne, an der Grundplatte lösbar angebrachte Klingen, die in Rillen in der Grundplatte angeordnet sind, die jeweils in einem Teilabschnitt eine Vertiefung aufweisen, in die vom unteren Rand der Klingen vorstehende Haltelaschen eingreifen. Die bekannte Vorrichtung ermöglicht keine Stabilisierung einer Wellenform. Die entstehenden Wellen werden vielmehr flachgedrückt und durch Vernähung röhrenförmig auf der Ausgangsbahn fixiert.

Eine weitere Einrichtung zum Ausbilden von Längsfalten in einer laufenden Bahn wird in der deutschen Patentschrift DE 3611134 C2 beschrieben. Hier wird ein sehr elastisches, durch Befeuchtung fast plastisch verformbares Papier für z.B. Zigarettenfilter zwischen einer großen Anzahl von ineinander kämmenden Walzen langsam von der Mitte nach außen wellenförmig eingezogen. Diese sehr aufwendige Maschine ist in der dargestellten Anwendungsform jedoch nur für abschnittsweise Wellenbildung gedacht, über die gesamte Bahn laufende Wellen sind wegen der ständigen wechselseitigen Bahnkrümmung nicht herstellbar.

In der deutschen Patentschrift DE 2827495 C2 schließlich wird eine Vorrichtung zum Fördern und Zusammenlegen für eine Materialbahn beschrieben, wobei im wesentlichen der Transport der Bahn bei gleichzeitigem trichterförmigen Einzug mittels strömender Luft Gegenstand der Erfindung ist. Eine exakt wellenförmige Ausbildung der Falten in der Bahn ist mit der bekannten Vorrichtung nicht möglich.

Der vorliegenden Erfindung liegt somit die Aufgabe zugrunde, einen absorbierenden Artikel anzugeben, welcher die Nachteile des Stands der Technik vermeidet. Diese Aufgabe löst die Erfindung durch den im unabhängigen Anspruch 1 angegebenen absorbierenden Artikel. Weitere vorteilhafte Ausgestaltungen, Aspekte und Details der Erfindung ergeben sich aus den abhängigen Ansprüchen, der Beschreibung und der Zeichnung.

Eine Vorrichtung zur Herstellung des erfindungsgemäßen Artikels weist ein Führungsbett auf, in das fächerförmige Nuten bzw. Rillen eingeschnitten sind, die bei einer vorzugsweise linear zunehmenden Tiefe schließlich zu einem wellenförmigen Querschnitt der gewünschten Abmessungen führen und in die von der Gegenseite entsprechend der zur Verfügung stehenden Höhe Niederhalter eintauchen. Die mittels der Vorrichtung erzeugten gewellten Bahnen können über die gesamte Bahnbreite dieselbe oder bei entsprechender Gestaltung des Führungsbetts auch unterschiedliche Wellenhöhe aufweisen. Es versteht sich für den Fachmann von selbst, daß das Führungsbett auch nur eine Rille aufweisen kann, wenn die gewünschte gewellte Bahn entsprechende Eigenschaften aufweisen soll. Dann ist auch nur ein Niederhalter notwendig. Die Niederhalter sind vorzugsweise stabförmig ausgebildet und vorzugsweise sowohl an dem dem Eintrittsende des Führungsbetts als auch an dem dem Austrittsende des Führungsbetts zugewandten Ende senkrecht zum Führungsbett frei verschiebbar. Die Niederhalter können an beiden Enden gehaltert sein oder ein freies, nicht geführtes oder gelagertes Ende aufweisen. Es ist von Vorteil, wenn die Niederhalter eine gewisse Elastizität aufweisen. Federstahl ist ein geeignetes Material für die Niederhalter. Die Niederhalter können in zumindest einer senkrecht zum Führungsbett verlaufenden Führungsfläche geführt sein. Drücken die Niederhalter zusätzlich durch kraftausübende Mittel mit einer vorgegebenen Kraft die Bahn gegen das Führungsbett, so wird der laufenden Bahn ein gewisser Einstelleffekt ermöglicht, welcher Spannungsunterschiede im Material durch die nicht immer geometrisch exakte Faltenbildung ausgleicht. Die vorgegebene Kraft auf die Niederhalter kann auch durch eine Vorspannung der Niederhalter ausgeübt werden. Zusäztliche kraftausübende Elemente können dann entfallen. Neben einem runden Querschnitt, der besonders bevorzugt ist, können die Niederhalter beispielsweise auch einen dreieckigen, rechtekkigen, halbrunden oder trapezförmigen Querschnitt aufweisen.

Besonders günstig ist es, wenn die freien Enden der Niederhalter im wesentlichen parallel zueinander abgebogen oder mit entsprechenden Ansätzen versehen sind, die in Bohrungen von zumindest einem endseitig gelegenem Führungselement aufgenommen werden. Es ist des weiteren von Vorteil, wenn alle Niederhalter bezüglich der Laufrichtung der Bahn vor bzw. nach dem Oberflächenbereich des Führungsbetts, welches die Rillen aufweist, aufgenommen sind. Die Niederhalter weisen vorzugsweise einen runden Querschnitt auf, wobei der Durchmesser beispielsweise von 0,1 bis 5 mm, vorzugsweise 1 bis 3 mm, insbesondere 2 mm, betragen kann. Der Abstand der Rillen kann z.B. 1 bis 10 mm, vorzugsweise 2 bis 4 mm, betragen.

Die Mittel zur Kraftausübung können beispielsweise eine Druckplatte sein, welche zwischen den Enden der Niederhalter auf diese einwirkt. Die Druckplatte kann zusätzlich mit zumindest einem Federelement versehen sein. Auch Gewichte, Druckluft, Vakuum oder Magnete können zur Kraftausübung eingesetzt werden. Werden Magnete verwendet, sind sowohl Permanent- als auch Elektromagnete geeignet, welche auf der der zu bildenden Bahn gegenüberliegenden Seite des Führungsbetts angeordnet sein sollten. Daß bei der Verwendung von Magneten die Niederhalter oder ein auf die Niederhalter einwirkendes Mittel, beispielsweise eine aufliegende Druckplatte, ferromagnetische Eigenschaften haben sollten, ist für den Fachmann klar.

Die mittels der Vorrichtung oder des Verfahrens erzeugten gewellten Bahnen aus dünnem Material müssen nach dem Austritt aus dem Führungsbett fixiert werden, damit die Wellenbildung dauerhaft bleibt. Eine Möglichkeit, die gewellte Bahn zu fixieren ist die Verbindung mit einem bahnförmigen Material (Trägerbahn) oder mehreren bahnförmigen Materialien (Trägerbahnen). Eine andere Möglichkeit der Fixierung besteht darin, die gewellte Bahn zwischen Prägewalzen dauerhaft plastisch zu verformen. Es ist weiterhin möglich, die gewellte Bahn durch Aufsprühen eines Verfestigungsmittels in der Wellenform zu fixieren. Wird eine Trägerbahn zur Stabilisierung der gewellten Bahn verwendet, so erfolgt die Verbindung zwischen den Bahnen vorzugsweise durch ein Haftmittel wie einen Klebstoff. Die Verbindung kann aber beispielsweise auch durch thermische Einwirkung oder Ultraschallschweißung erfolgen.

Gemäß einer weiteren Ausführungsform ist das Führungsbett im Querschnitt mit unterschiedlicher Wellenhöhe ausgebildet, wobei in der Regel in der Mitte eine maximale Wellenhöhe geformt wird, welche zu den Rändern des Führungsbetts hin abnimmt.

Es ist des weiteren von Vorteil, wenn das Führungsbett, quer zur Laufrichtung der Materialbahn gesehen, so ausgestaltet ist, daß die Rillen nicht alle auf gleicher Höhe beginnen. Ein Beginn der Rillen im Mittelbereich des Führungsbetts ist dabei besonders bevorzugt, wobei dann weiter stromabwärts beginnende Rillen in den Randbereichen angeordnet sind. Auch ist es für manche Ausführungsformen wünschenswert, wenn das Führungsbett so ausgebildet ist, daß nicht die gesamte Breite mit Rillen versehen ist, sondern im Mittelteil und/oder in Randbereichen des Führungsbetts keine Rillen vorgesehen sind.

Schließlich kann die Vorrichtung eine Einrichtung mit kämmenden Walzen zur Prägung der erzeugten gewellten Bahn aufweisen, wobei die Einrichtung mit kämmenden Walzen dem Führungsbett nachgeschaltet ist.

Die mit dem Verfahren und der Vorrichtung hergestellte gewellte Bahn kann vorteilhafterweise als Bestandteil des erfindungsgemäßen absorbierenden Artikels zur Aufnahme von Körperflüssigkeiten, wie beispielsweise einer Windel, einer Damenbinde oder einer Inkontinenzeinlage, eingesetzt werden. Ein entsprechender absorbierender Artikel weist üblicherweise eine bei Gebrauch dem Körper abgewandte flüssigkeitsundurchlässige Abdecklage, eine bei Gebrauch dem Körper zugewandte flüssigkeitsdurchlässige Abdecklage und einen zwischen der flüssigkeitsdurchlässigen und der flüssigkeitsundurchlässigen Abdecklage angeordneten Saugkörper auf. Der erfindungsgemäße absorbierende Artikel ist dadurch ausgezeichnet, daß die flüssigkeitsdurchlässige Abdecklage und/oder der Saugkörper zumindest in Teilbereichen eine gewellte ("plissierte") Form aufweisen, wobei Bahnen verwendet werden können, die mittels dem Verfahren oder der Vorrichtung in Falten gelegt wurden. Gegenüber wie herkömmlich mittels Prägewalzen hergestellten gewellten Bahnen haben die hergestellten Bahnen den Vorteil eines angenehmen Tragekomforts bzw. einer verbesserten Saugleistung, da das bearbeitete Material beim Formungsvorgang praktisch nicht verfestigt wird.

Die Erfindung wird nachfolgend anhand der Zeichnungen näher erläutert. Es zeigen
- Fig. 1: eine perspektivische, teilweise aufgeschnittene Ansicht einer Maschine zur Herstellung einer gewellten Bahn aus sehr elastischem Material, die mit einer Trägerbahn verbunden wird,
- Fig. 2: eine perspektivische Draufsicht auf die gewellte Bahn, die zugleich der Form des Führungsbetts in einer bevorzugten Ausführung darstellt,
- Fig. 3: eine perspektivische Darstellung einer alternativen, in der Mitte beginnenden Wellenausbildung,
- Fig. 4: eine perspektivische Draufsicht auf eine Bahn bzw. ein Führungsbett mit nur teilweiser Wellenbildung,
- Fig. 5: einen Querschnitt durch drei Sektionen des Führungsbetts am Anfang, in der Mitte und am Ende mit einer schematischen Darstellung der eingezogenen Bahn,
- Fig. 6: verschiedene theoretische Ausformmöglichkeiten der gewellten Bahn mit unterschiedlichen Einzugsfaktoren,
- Fig. 7: materialtechnische Betrachtungen für vorzugsweise zu verarbeitende Bahnen,
- Fig. 8: eine Seitenansicht des Führungsbettendes mit einer Verbindungseinrichtung sowie die Fixierung einer Andruckmöglichkeit der Niederhalter mittels Pneumatikzylinder und Kniehebel,
- Fig. 9: eine teilweise aufgeschnittene perspektivische Ansicht des Führungsbetts und der Niederhalter, auf welche hier durch getrennt ansteuerbare Elektromagnete jeweils abschnittsweise veränderliche Kräfte aufgebracht werden können,
- Fig. 10: eine Ansicht der beiden Walzen der Verbindungsstation mit den in die gewellte Walze eingreifenden Niederhaltern und streifenförmigem Leimauftrag,
- Fig. 11: eine schematische Darstellung von nachgeschalteten Prägewalzen, welche die gewellte Bahn an den Wellenspitzen zur Fixierung durch Pressen verformen,
- Fig. 12: eine schematische Darstellung der Aufbringung einer zweiten Bahn mit der anschließenden Preßstation zur Fixierung der Klebeverbindung,
- Fig. 13: eine schematische Darstellung einer weiteren bevorzugten Ausführung zur Verklebung der zweiten Bahn, und
- Fig. 14 bis: 21 schematische Darstellungen von absorbierenden Artikeln gemäß der Erfindung.

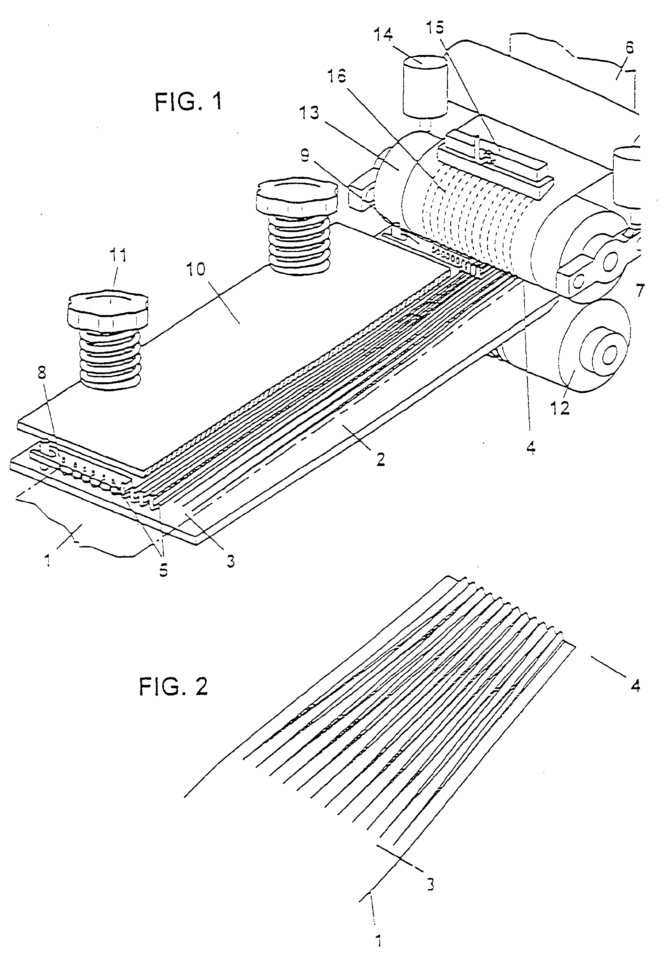

Die zu wellende Bahn 1 tritt gemäß Fig. 1 an der Vorderkante 3 des Führungsbetts 2 in die Wellvorrichtung ein, wird durch die stabförmig ausgebildeten Niederhalter 5 in das Führungsbett gedrückt und verläßt dies am Austrittsende 4 in gewellter Form. Die Niederhalter sind in einem vorderen Abstandshalter 8 und einem hinteren Abstandshalter 9 zusammengefaßt, so daß ein gegenseitiger Abstand konstant gehalten wird und sie werden bei der in Fig. 1 gezeigten Ausführungsform von einer über Einstellschrauben 11 mittels Federn beaufschlagten Druckplatte 10 mit einer definierten Kraft angedrückt. In Richtung auf das Führungsbett sind die Niederhalter frei verschiebbar gelagert.

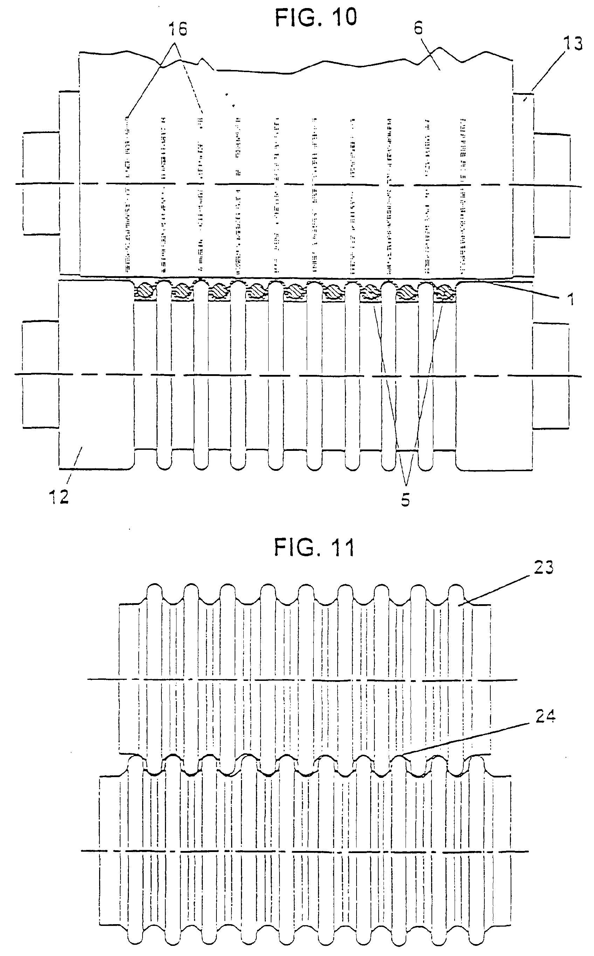

Unmittelbar nach dem Verlassen der Führungsbahn 2 erfolgt das Zusammenführen der gewellten Bahn mit der Trägerbahn 6, das über eine glatte Walze 13 und durch eine im Querschnitt profillierte Walze 12, wie auch in Fig. 10 als Ansicht dargestellt, bewirkt wird. Die für die anschließende Verklebung der Schichten erforderliche Anpreßkraft wird hier mittels zweier Pneumatikzylinder über Hebel aufgebracht.

Zum Verbinden der Trägerbahn mit der gewellten Bahn ist in diesem Falle eine Lösung mit linienförmigen Klebstoffauftrag 16 mittels einer Mehrfach-Leimauftragsdüse 15 dargestellt. Durch die besondere Form und Befestigung der stabförmigen Niederhalter, deren Enden bis in die profillierte Walze 12 reichen, was auch aus Fig. 10 ersichtlich ist, wird ein Zurückfedern der ausgeformten Wellenbahn vor dem Verkleben verhindert.

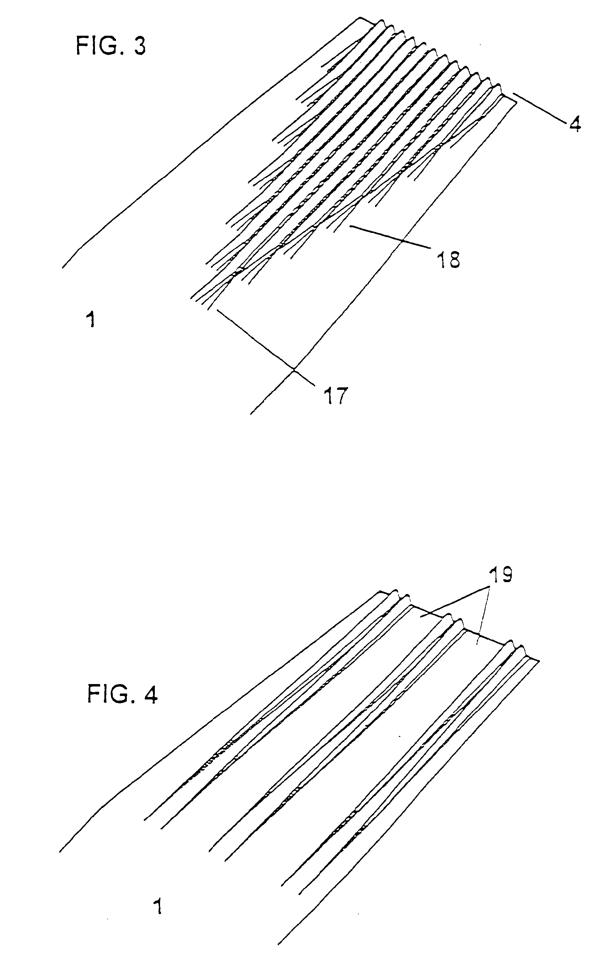

Die gewellte Bahn 3 in Fig. 2 entspricht im wesentlichen dem ebenen Führungsbett, man sieht ein geradliniges Ansteigen der Wellen, welches eine einfache Herstellung der Ausformeinrichtung ermöglicht. Um bei weniger dehnbarem Material die lokalen Spannungen zu verringern, kann das Führungsbett vorteilhaft entsprechend Fig. 3 ausgebildet werden, wo die Wellenbildung in der Mitte der Bahn beginnt und weiter am Rand liegende Wellen erst später ausgeformt werden. Allerdings ist bei einer solchen Ausführung der Einbau der Niederhalter mit erhöhtem Aufwand verbunden, da diese auf unterschiedlich langen Strecken die volle Wellenhöhe ausformen müssen und verschiedene Neigung gegen das Führungsbett aufweisen.

Für Bahnen, die entsprechend ihrer Anwendung nicht über die ganze Breite gewellt sein sollen, läßt sich das Führungsbett gemäß Fig. 4 auch für nur wenige Wellen mit dazwischenliegenden ebenen Führungsbettabschnitten 19 ausbilden. Im Extremfall kann das Führungsbett auch nur eine Rille aufweisen, wie vorstehend bereits angedeutet wurde.

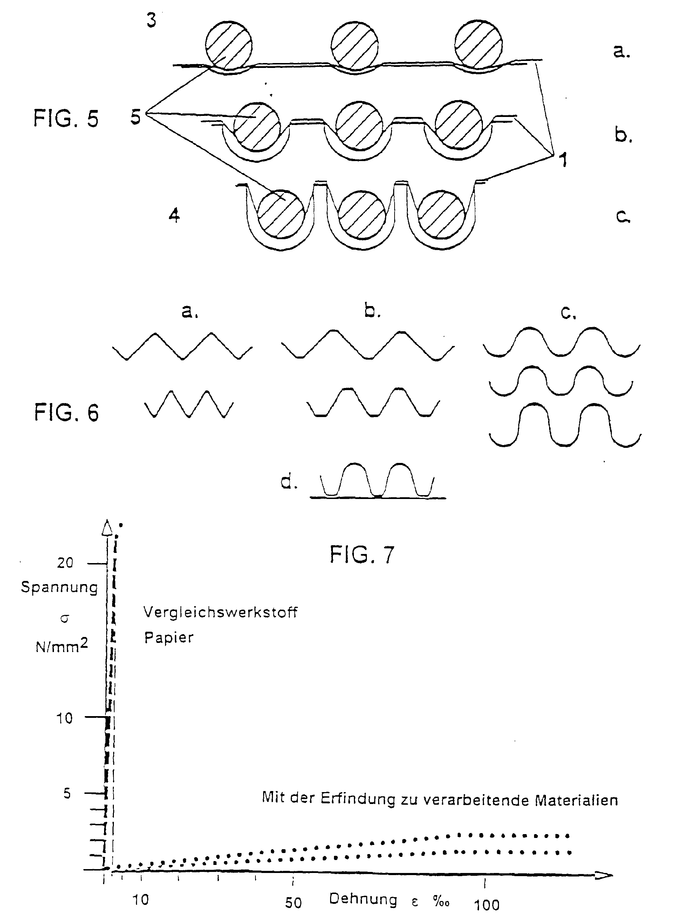

Die Ausgestaltung von Führungsbett und Niederhaltern kann zu einem nicht über die gesamte Länge der Wellenbildungsstrecke gleichmäßigen Einzugsverhalten führen, was in Fig. 5 schematisch dargestellt ist. Mittels eines Berechnungsprogramms kann der Einzugsfaktor für die zu wellende Bahn an jeder Stelle des Führungsbettes ermittelt und optimiert werden. Am Beginn der Faltenbildung, dargestellt unter a, und am Ende gemäß Bild c, wird der ideale Wert erreicht, während etwa in der Mitte des Führungsbetts entsprechend Bild b nur ca. 92 Prozent des Materials eingezogen werden, was jedoch durch die Dehnbarkeit und geringe Biegesteife der Bahn ausgeglichen wird.

Je nach Aufgabengebiet sind die unterschiedlichsten Wellenformen gemäß Fig. 6 theoretisch herstellbar, jedoch würden die eckig ausgeformten Wellen nach a und b eine chemische oder physikalische Behandlung, beispielsweise durch Einsprühen mit einem Verfestigungsmittel, thermoplastische Verfestigung oder sehr starkes plastisches Ausprägen erfordern, um den gezeigten Querschnitt beizubehalten. Die Einzugswerte schwanken zwischen 1,41 bei 45 Grad Dreieckswelle bzw. 1,32 für die Trapezform mit einem Drittel Geradenabschnitt und dem Wert 2 bei 60 Grad Steigung der Welle bzw. 1,66 beim Trapez.

Für die bevorzugte Ausführungsform c mit Halbkreisen und mehr oder weniger langen Geradenabschnitten beträgt der Materialeinzug zwischen 1,45 bei kurzen Geradenstücken mit 50 Grad Neigung und Werten größer als 2 bei fast senkrechten Geradenanteilen. Eine Sonderstellung nehmen die beiden aneinandergereihten Halbkreise ein, hier beträgt der Einzugsfaktor genau π/2, also 1,57. Diese Wellenform ist auch von der Ausgewogenheit der inneren Spannungen und der eBelastbarkeit durch äußere Kräfte ohne zusätzliche, stabilisierende Maßnahmen vorteilhaft. Bei Verklebung mit einer Trägerbahn wird sich zwangsweise - je nach Verbindungsverfahren - eine gewisse Abplattung der Wellen einstellen, so daß der praktisch erzeugte Wellenquerschnitt die Form d annimmt.

Um die Maschine durchlaufen zu können, sollte die zu verarbeitende Bahn bestimmte physikalische Eigenschaften aufweisen: geringe Biegesteifigkeit und hohe Dehnbarkeit. Diese Forderung wird beispielsweise von faserigen Stoffen geringer Dichte und Kunststoff-Folien erfüllt. In Fig. 7 ist eine Spannungs-Dehnungs-Kurve für verschiedene Materialien angegeben. Im Gegensatz zu Papier, bei dessen Herstellung auf möglichst gerine Deformierbarkeit und hohe Festigkeit Wert gelegt wird, besitzen solche Stoffe eine gewisse Verformbarkeit, verbunden mit einer meist nicht genau definierten Streckgrenze und hoher Bruchdehnung. Vorzugsweise sind die verarbeiteten Materialbahnen zumindest in Querrichtung elastisch.

Im Falle der Wellenausbildung wird gegen Ende des Ausformvorgangs rechnerisch von der Bahn in Querrichtung eine etwa 8 % längere Strecke als in der Mitte des Vorgangs gefordert, was jeder der bisher untersuchten Stoffe problemlos überstand, da dies allenfalls zu einer minimalen, bleibenden Deformation führt, die im vorliegenden Anwendungsfall noch nicht einmal unerwünscht ist. Folglich ist die Maschine mit dem einfach ausgebildeten Führungsbett zur Wellenbildung in dünnen Bahnen insbesondere für alle Werkstoffe, welche ca. 10 % Dehnung und mehr ohne Bruch erreichen, besonders geeignet. Obwohl keine Obergrenze für die Dehnung besteht, sind Materialien bevorzugt, welche eine Dehnung von 10 bis 150 % zulassen.

In der nachfolgend angegebenen Tabelle 1 sind einige Beispiele von Materialien bzw. Materialkombinationen angegeben, welche mit der Vorrichtung und mittels des Verfahrens zur Herstellung gewellter Bahnen verarbeitet werden können. Die hergestellten gewellten Bahnen werden auch als "plissierte" Bahnen bezeichnet und das dazu verwendete Material entsprechend als "plissierbares" Material.

**Tabelle 1**

| **Materialien** | **Dimensionen** | **Kardenvlies** | **Spinnvlies** | **Laminat Kardenvlies/ Lochfolie** | **Laminat Spunbond/ Lochfolie** |
|---|---|---|---|---|---|
| **Flächengewicht** | **g/m**^{**2**} | **18** | **16** | **43** | **42** |
| **Dicke** | **mm** | **0,18** | **0,13** | **0,92** | **0,50** |
| **Festigkeiten, trocken** | | | | | |
| **längs** | **N/50 mm** | **40** | **30** | **55** | **13** |
| **quer** | **N/50 mm** | **18** | **20** | **19** | **6** |
| **Dehnung, trocken** | | | | | |
| **längs** | **%** | **50** | **50** | **35** | **34** |
| **quer** | **%** | **90** | **60** | **150** | **78** |
| **Reibung, trocken** | | | | | |
| **Innen(Folien)-seite** | ***µ*D** | **0,65** | **0,33** | **0,41** | **0,30** |
| **Außen(Vlies)-seite** | ***µ*D** | **0,65** | **0,33** | **0,41** | **0,93** |

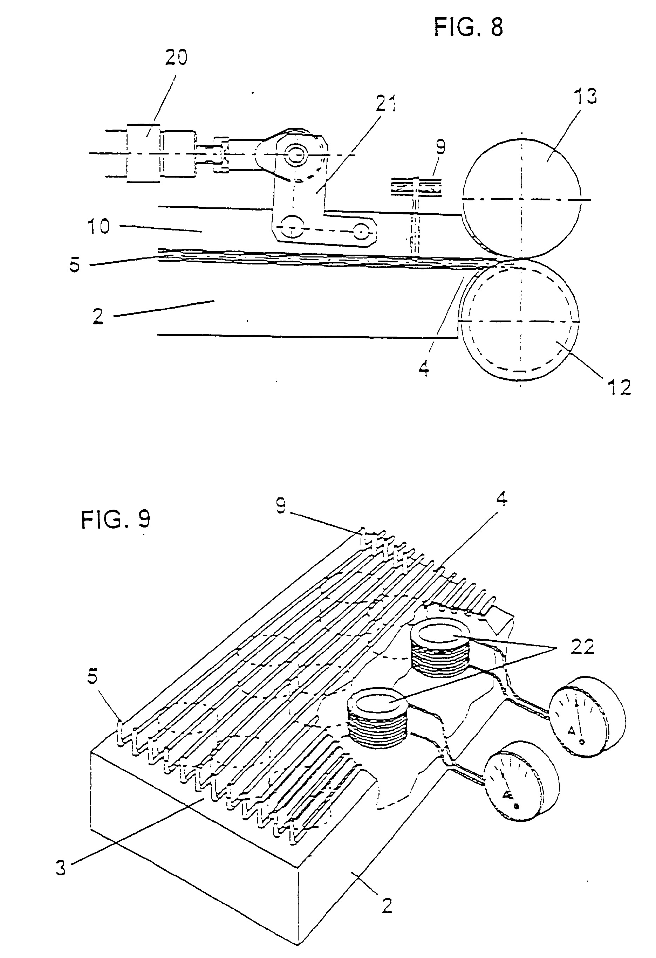

Günstig für die gleichmäßige Wellenausbildung mit der Vorrichtung ist eine definierte Druckkraft auf die Bahn, welche durch die Niederhalter aufgebracht wird. Diese kann durch Gewichte, Federkraft (siehe Fig. 1) oder auch während des Betriebs steuerbar entsprechend Fig. 8 über Pneumatik- oder Hydrulikzylinder 20 geschehen, wobei wegen der garingen erforderlichen Kräfte und weniger Leckage einem Pneumatikzylinder der Vorzug zu geben ist. Zur Einsparung von Bauhöhe kann dieser z.B. längs angeordnet werden und über Kniehebel 21 auf die Andrückplatte 10 wirken. Nach theoretischen Untersuchungen und praktischen Versuchsergebnissen hat sich je nach verarbeitetem Material eine Linienlast von 0,04 bis 0,06 Ncm⁻¹ für Bahngeschwindigkeiten bis 150 m/min günstig erwiesen. Die Gesamtauflagekraft erreicht damit bei einer Länge des Führungsbetts von 300 mm etwa 20 N. Bei Veränderung des Flächengewichts und/oder der Biegesteifigkeit des Materials ist auch eine Kraft von beispielsweise 0,01 bis 0,1 Ncm⁻¹, vorzugsweise 0,02 bis 0,08 Ncm⁻¹ geeignet. Die Lösung mit Pneumatikzylinder hat neben der Steuerbarkeit der Niederhalterkräfte auch den Vorteil, daß zum Einfädeln der Bahn die Einrichtung pneumatisch angehoben und entlastet werden kann. Mit geeigneten Materialien können problemlos Bahngeschwindigkeiten von bis zu 250 m/min erzielt werden.

Fig. 8 zeigt eine Seitenansicht der bis in die profillierte Walze 12 reichenden Niederhalterstäbe 5 mit der über Klemmschrauben einstellbaren hinteren Halterung 9.

Um eine noch individuellere Kraftverteilung in den Niederhaltern zu erzielen, können in das Führungsbett gemäß der in Fig. 9 gezeigten bevorzugten Ausführungsform der Vorrichtung Elektromagnete 22 eingelassen werden, die je nach Erfordernissen gruppenweise oder einzeln zu beaufschlagen sind, wodurch sich eine besonders feinfühlige Einflußnahme auf die Wellenausbildung ergibt. Die ferromagnetischen Niederhalterstäbe 5 werden von Elektromagneten mit bis zu 0,1 Nmm⁻² angezogen, d.h. bei einer Stabbreite von 2 mm genügt entweder ein sehr geringer Strom oder eine entsprechend verteilte geringe Anzahl von Einzelmagneten, um den erforderlichen Anpreßdruck zu erzeugen. Diese Lösung hat den Vorteil, daß trotz eines eventuell unterschiedlichen Verschleißes der Niederhalterstäbe dennoch eine gleichmäßige Wellenausbildung möglich ist, ohne daß Nachstellarbeiten erforderlich werden.

Wegen der Instabilität des verarbeiteten Materials würden die ausgeformten Wellen ohne zusätzliche Vorkehrungen wie etwa einer Verbindung mit einer Trägerbahn, einer thermoplastischen Veränderung oder einem Fixieren mittels eines aufgesprühten Verfestigungsmittel nach Verlassen der Formeinrichtung in den ebenen Zustand zurückgefedert.

Wenn eine Trägerbahn zur Stabilisierung verwendet wird, kann diese dabei aus dem gleichen Material wie die gewellte Bahn oder auch aus einem anderen Material bestehen. Die Verbindung mit einer Trägerbahn erfolgt vorzugsweise, wie in den Fig. 1 und 10 gezeigt, durch Verkleben einer glatten Bahn 6 mit den Spitzen (Maxima bzw. Minima) der ausgeformten Wellenbahn 1. Die Wellen werden während dieses Vorgangs in einer entsprechend dem Abstand der Niederhalter 5 mit Nuten versehenen Walze 12 und den Stabenden der Niederhalter zwangsweise gehalten, während eine von oben z.B. über Pneumatikzylinder angedrückte Walze die zweite Bahn 6 zuführt, auf die mittels einer Mehrfachdüse jeweils im Bereich der Wellenberge linienförmig Klebstoff 16 aufgebracht wird.

Das Verbinden von gewellter Bahn mit Trägerbahn gemäß Fig. 1 durch linienförmigen Klebstoffauftrag 16 ist nur eine der hierfür geeigneten Möglichkeiten. Auch der Einsatz von Radbzw. Siebdruck-Leimauftragsgeräten ist denkbar.

Da die Vorrichtung vorzugsweise zur Verarbeitung von Bahnen aus synthetischen Folien oder Vliesen eingesetzt werden kann, kann durch Einbringen thermischer Energie beispielsweise über die gewellte Transportwalze 12 ein Verschweißen der gebildeten Wellenbahn mit der Trägerbahn erfolgen.

Einen ähnlichen Verbindungseffekt bewirkt das Zusammendrücken der Bahnen im Wellental der aufliegenen gewellten Bahn z.B. durch einen mit Ultraschall beaufschlagten Preßschuh oder eine Preßwalze.

Um die ausgebildete Wellenform auch ohne zusätzliche Trägerbahn zu fixieren, kann die gewellte Bahn vorzugusweise gemäß Fig. 11 zwischen Prägewalzen 23 mit wellenförmigem Querschnitt in den Wellenspitzen 24 plastisch verformt werden. Dieser Effekt kann außerdem durch thermische oder chemischpyhsikalische Einwirkung, wie etwa dem Aufsprühen eines Verfestigungsmittels, erreicht oder verstärkt werden.

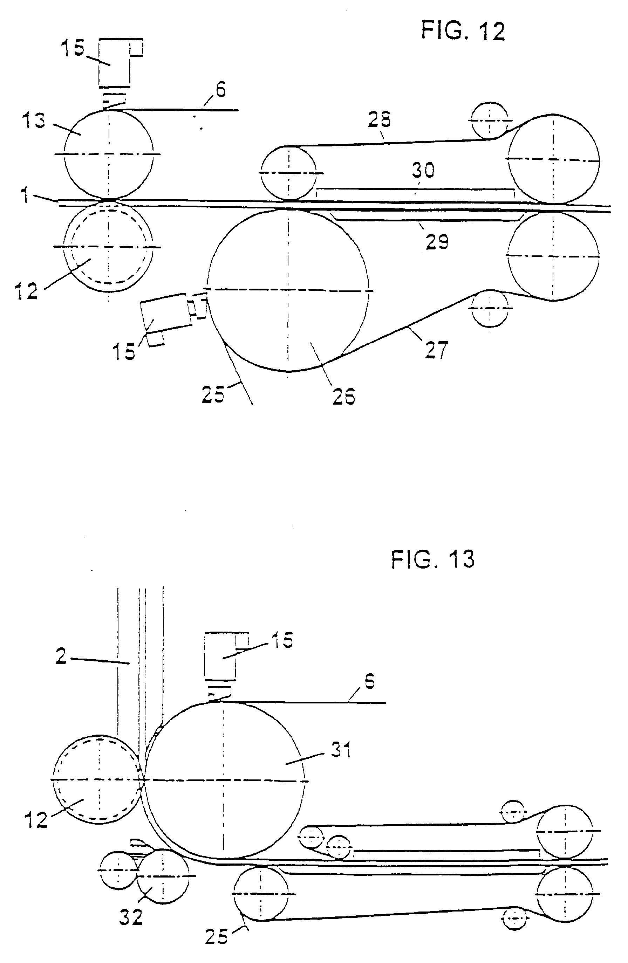

Eine weitere vorteilhafte Ausführung besteht gemäß Fig. 12 darin, die der Trägerbahn 6 gegenüberliegende Seite der Wellenbahn ebenfalls mit einer weiteren Materialbahn 25 zu verbinden. Die Materialbahn 25 wird auch als Deckbahn bezeichnet. Zu diesem Zweck kann beispielsweise diese Bahn mittels einer Walze größeren Durchmessers 26 und einem Riemensystem 27 zugeführt werden, wobei mittels einer Auftragsvorrichtung 15 streifenförmig Klebemittel aufgebracht wird. In einer vorteilhaften Ausführung kann durch ein zweites Riemensystem 28 und eine Andrückvorrichtung 30, welche den Anpreßdruck gleichmäßig verteilt, die Verklebung mit der Wellenbahn 1 erfolgen, wobei die Unterstützung 29 dem Anpreßdruck entgegenwirkt.

Gemäß Fig. 13 wird die aus dem Führungsbett 2 austretende Wellenbahn mittels einer gewellten Walze 12 gegen eine glatte Walze 31 größeren Durchmessers gedrückt, wobei sie mit der Trägerbahn 6, auf die beispielsweise vorher mittels einer geeigneten Vorrichtung 15 streifenförmig Klebstoff aufgebracht wurde, verbunden wird.

Die Krümmung der Walze 31 ist so gewählt, daß die Wellenbahn lediglich geringfügig elastisch verformt wird und nach dem Verlassen der Walze ihren ursprünglichen Querschnitt wieder einnimmt. Mittels eines im Prinzip bekannten Leimwerks 32 kann in einem Bereich der Walze 31 auf die Wellenspitzen Klebstoff aufgetragen werden. Durch eine anschließende Anpreßstrecke mit kontrolliertem Anpreßdruck, wie sie unter Fig. 12 beschreiben ist, wird die Verbindung gefestigt.

Die Erfindung betrifft einen absorbierenden Artikel, welcher zur Aufnahme von Körperflüssigkeiten geeignet ist. Derartige Artikel sind beispielsweise Artikel für die Frauenhygiene, wie Damenbinden, sowie Windeln, Inkontinenzeinlagen oder dergleichen.

Die vorgenannten Hygieneartikel sind in verschiedensten Ausführungsformen bekannt. Diesen ist eine rückseitige, flüssigkeitsdurchlässige Abdecklage, eine vorderseitige, flüssigkeitsdurchlässige Abdecklage und ein zwischen diesen beiden Lagen angeordneter Saugkörper gemeinsam.

Die rückseitige Abdecklage ist üblicherweise aus einer dünnen Polyethylen-Folie gebildet. Für die vorderseitige, flüssigkeitsdurchlässige Abdecklage werden meist Vliesstoffe eingesetzt. Auch die Verwendung gelochter Folien ist bekannt.

Der Saugkörper besteht in der Regel aus Zellstoff-Flocken oder einer luftgelegtten Faserbahn - einem sogenannten Air-Laid-Material.

Neben der grundsätzlichen Saugleistung des Saugkörpers bilden produkttechnische Eigenschaften der vorderseitigen - also dem Körper des Trägers zugewandten - Abdecklage, wie taktile Weichheit, Ansaugzeit bezüglich der Körperflüssigkeit, deren Verteilung über den Hygieneartikel und die Rücknäßeigenschaften eine entscheidende Rolle. Die üblicherweise für die körperseitige Abdecklage verwendeten Vliesstoffe sind in ihren diesbezüglichen Eigenschaften weitgehend vergleichbar.

Durch die erfindungsgemäße Faltenlegung der vorderseitigen Abdecklage wird ein Oberflächeneffekt erzielt, der am ehesten mit dem Ausdruck "Plissierung" umschrieben werden kann.

Durch die Falten werden gleichzeitig Längskanäle an der Oberfläche des Hygieneartikels zur Verbesserung der Flüssigkeitsverteilung in Längsrichtung und Barrieren gegen eine Flüssigkeitsausbreitung in Querrichtung geschaffen. Dies verbessert erheblich die Auslaufsicherheit des Hygieneartikels.

Weiterhin wird durch die Falten in erheblichen Bereichen der Abdecklage ein Abstand zwischen diesen und dem darunter befindlichen Saugkörper erzielt, der das Ansaug- bzw. Eindringverhalten der Körperflüssigkeit in den Saugkörper und gleichzeitig die Rücknäßeigenschaften verbessert. Auch wird der visuelle Eindruck nach einer Beaufschlagung z.B. durch Menstruationsflüssigkeit, verbessert, da die erhabenen Faltenbereiche nicht mit dem flüssigkeitsgetränkten Saugkörper in Verbindung stehen. Nicht zuletzt wird durch die Nachgiebigkeit der Falten die Weichheit der dem Körper zugewandten Oberfläche des Hygieneartikels beeinflußt, was den Tragekomfort und den natürlichen Griff verbessert.

Durch die Ausbildung eines Teils des Saugkörpers in plissierter Form kann ein entsprechender Effekt hinsichtlich einer Verbesserung der Flüssigkeitsverteilung ebenfalls erreicht oder in Kombination mit einer plissierten Abdecklage sogar noch verbessert werden. Geeignete Materialien für den plissierten Saugkörper sind hydrophile Vliese, wie hydrophile Kardenvliese oder hydrophile Spinnvliese mit Flächengewichten von 6 bis 80 g/m², insbesondere 10 bis 30 g/m², luftgelegte Faserbahnen und Laminate.

Für die plissierte Abdecklage können hydrophobe Vliese eingesetzt werden, wie hydrophobe Kardenvliese und hydrophobe Spinnvliese. Das Flächengewicht entsprechender Materialien liegt wiederum vorzugsweise im Bereich von 5 6 bis 30 80 g/m², insbesondere 10 bis 20 g/m².

Wenn gemäß einer bevorzugten Ausführungsform die dem Saugkörper zugewandten Fußbereiche der Falten auf dem Saugkörper oder einer zwischen diesem und der vorderseitigen Abdecklage angeordenten Trägerlage fixiert sind, werden die Falten so stabilisiert, daß die von ihnen hervorgerufenen positiven Effekte auch nach einer Lagerung im gepreßten Zustand und über eine längere Tragezeit erhalten bleiben.

Die Fußbereiche der Falten können durch eine Verklebung (Klebstoff-Streifen) oder Verschweißung auf dem Saugkörper oder der Trägerlage auf einer Breite (Fußbreite F) von vorzugsweise 0,2 bis 10 mm fixiert werden.

Die Trägerlage kann dabei die funktionellen Eigenschaften der Falten etwa hinsichtlich Flüssigkeitsweiterleitung und Rücknässung verbessern.

Die Fußbereiche zweier benachbarter Falten können mit einem gegenseitigen Abstand (A) von 1 bis 20 mm fixiert sein und die Abstehhöhe (II) der Falten in unbelastetem Zustand kann ebenfalls 1 bis 20 mm betragen.

Gemäß einer bevorzugten Ausführungsform können mehrere Falten über die gesamte Breite der Saugkörperoberfläche verteilt vorgesehen sein. Damit wird eine maximale Produktverbesserung erzielt. 5 bis 25 Falten über die gesamte Breite haben sich als besonders günstig erwiesen. Schließlich können mehrere, vorzugsweise 2 bis 10 Falten jeweils in auf eine Teilbreite der Saugkörperoberfläche begrenzten, randseitigen und/oder mittigen Streifen vorgesehen sein.

Der erfindungsgemäße absorbierende Artikel wird des weiteren anhand der beigefügten Zeichnung näher erläutert.

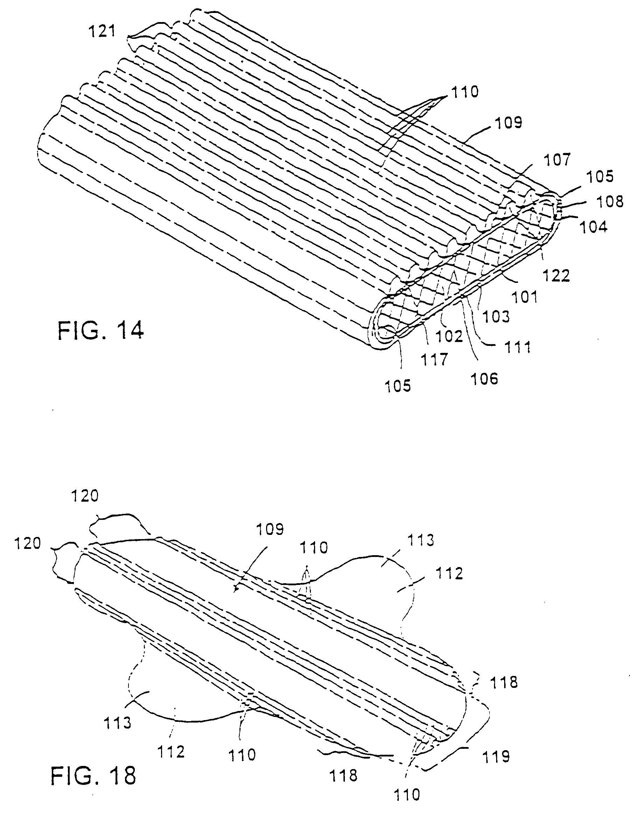

Wie aus den beigefügten Figuren 14 bis 20 deutlich wird, weisen die verschiedenen. Ausführungsformen der gezeigten Damenbinde einen in wesentlichen Punkten übereinstimmenden Grundaufbau auf. So ist als rückseitige, flüssigkeitsundurchlässige Abdecklage eine Wäscheschutzfolie 101 vorgesehen, die dem als Zellstoff-Flockenkissen ausgebildeten Saugkörper 102 im Bereich seiner Rückseite 103 - also der der Trägerin im angelegten Zustand der Damenbinde körperabgewandten Seite-unterliegt. Die Wäscheschutzfolie 101 greift ferner mit ihren Längskanten 104 um den Seitenrand 105 des Saugkörpers 102 herum.

Auf der vorderseitigen Oberfläche 106 verläuft eine flächig aufgelegte Trägerfläche 107, bei der es sich beispielsweise um eine Vliesstofflage handeln kann. Auch die Trägerlage 107 greift mit ihren Längskanten 108 um den Seitenrand 105 des Saugkörpers 102 herum und überlappt mit den Längskanten 104 der Wäscheschutzfolie 101. Im Überlappungsbereich sind diese beiden Teile in üblicher Weise miteinander verklebt.

Auf der Trägerlage 107 ist eine vorderseitige, flüssigkeitsdurchlässige Abdecklage 109 vorgesehen, die in noch näher zu erläuternder Weise mit Falten 110 versehen ist. Bei den in Fig. 14, 15 und 17 gezeigten Ausführungsformen der Damenbinde handelt es sich um eine sogenannte "Volleinschlagbinde", bei der die Abdecklage 109, die beispielsweise aus einem Polypropylen-Kardenvlies gebildet ist, um den Saugkörper 102 die Trägerlage 107 und die Abdecklage 109 auf der Rückseite 103 herumgeschlagen und mittig durch eine Klebeverbindung 111 fixiert.

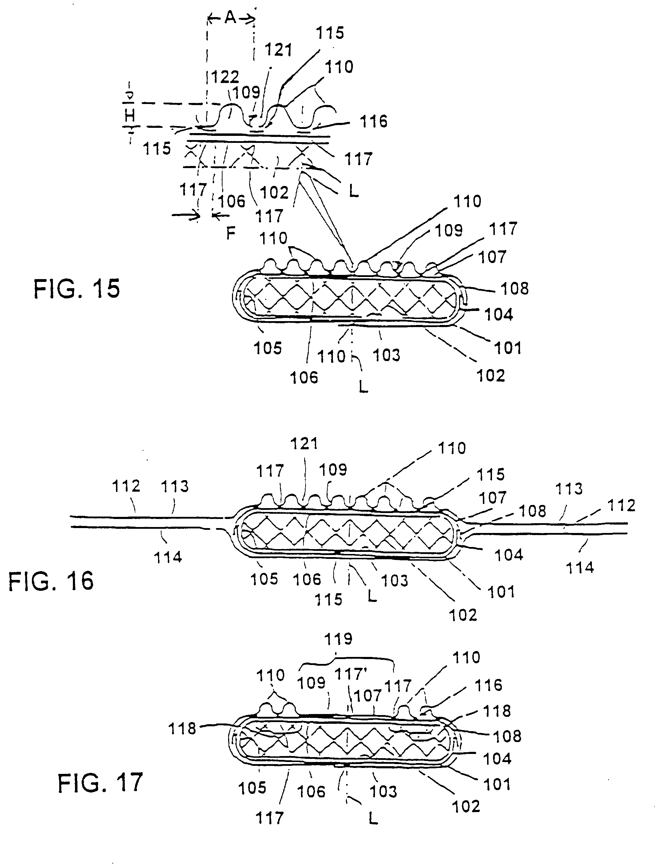

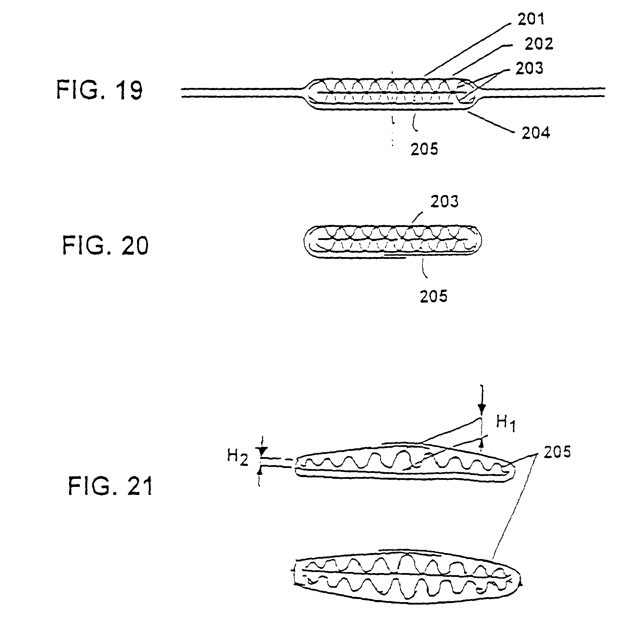

Bei den in Fig. 16 und 18 gezeigten Ausführungsformen sind seitliche Flügel 112 vorgesehen, die durch seitlich überstehende, miteinander randseitig verbundene Lappen 113, 114 der vorderseitigen Abdecklage 109 bzw. einer rückseitigen Decklage 115 gebildet sind. Die Decklage 115 kann aus Vliesstoff gebildet sein, wie dies insbesondere bei der in Fig. 16 gezeigten Ausführungsform der Fall ist, wo eine eigene Wäscheschutzfolie 101 vorgesehen ist. Die Decklage kann aber auch unter Weglassung der Wäscheschutzfolie 101 aus flüssigkeitsundurchlässigem Material, beispielsweise Polyethylen-Folie gefertigt sein, um somit die Wäscheschutzfunktion zu übernehmen.

Im folgenden werden die unterschiedlichen Faltenkonfigurationen, wie sie in den einzelnen Ausführungsformen der gezeigten Damenbinden verwendet werden, näher erläutert.

Die Falten 110 werden durch einen geeigneten Faltenleger in der Binden-Herstellungsmaschine so aufgebracht, daß die Fußbereiche 116 mit einem Abstand A in Querrichtung der Binde auf der Trägerlage 107 zu liegen kommen, der im Bereich zwischen 1 und 20 mm liegt. Als besonders praktikabel hat sich ein Abstandsbereich von 3 bis 5 mm herausgestellt. Die Fußbereiche 116 werden durch längsverlaufende Klebstoff-Streifen 117, die beispielsweise aus einem Sprühkleber gebildet sind, auf der Trägerlage 107 fixiert. Zwischen jeweils einander benachbarten Fußbereichen 116 steht die Falte 110 nach oben ab, wobei eine Abstehhöhe H im Bereich von 1 bis 20 mm denkbar ist. Als besonders praktikabel haben sich wiederum Werte für die Abstehhöhe herausgestellt, die im Bereich zwischen 3 und 5 mm liegen. Die durch die Klebstoff-Streifen 117 definierte Fußbreite F kann je nach Abstand A und Abstehhöhe H zwischen 0,2 und 10 mm betragen, wobei darauf zu achten ist, daß die Fußbreite F maximal die Hälfte des Abstands A beträgt. Bei einem Abstand A und einer Abstehhöhe H von 3 bis 5 mm haben sich Fußbreiten von 0,5 bis 1 mm bewährt.

Bei den in Fig. 14 bis 16 gezeigten Ausführungsbeispielen sind acht über die gesamte Breite der Saugkörperoberfläche 102 gleichmäßig verteilte Falten 110 vorgesehen, wobei mit einem Faltenabstand A von ca. 6 mm gearbeitet wird.

Bei der in Fig. 17 gezeigten Ausführungsform sind in zwei randseitigen Streifen 118 jeweils zwei in Längsrichtung der Damenbinde verlaufende Falten 110 vorgesehen, die wiederum einen Abstand A und eine Abstehhöhe H von ca. 6 mm aufweisen. In dem zwischen den beiden randseitigen Streifen 118 verbleibenden Mittenstreifen 119 liegt die flüssigkeitsdurchlässige Abdecklage 109 plan auf der Trägerlage 107 auf und ist durch einen mittigen Klebstoff-Streifen 117 zusätzlich it dieser verbunden. Auch eine vollflächige, flüssigkeitsdurchlässige Verklebung mit geringen Klebstoffmengen ist möglich.

Bei der in Fig. 18 skizzierten Ausführungsform einer Damenbinde ist zusätzlich zu den Falten 110 in den beiden randseitigen Streifen 118 symmentrisch zur Mittelebene L eine weitere Faltenkonfiguration mit drei Falten 110 vorgesehen, wobei faltenlose Streifen 120 zwischen den mittigen Falten 110 und den randseitigen Falten 110 verbleiben.

Wie aus den Querschnitten gemäß Fig. 15 bis 17 deutlich wird, bilden einerseits die auf der Außenseite der Falten 110 zwischen diesen liegenden Rinnen 121 und andererseits die unter den Falten 110 zwischen den verklebten Fußbereichen 116 liegenden Röhren 122 Kanäle zur Verteilung der die Damenbinde beaufschlagten Körperflüssigkeiten in Längsrichtung der Binde.

Es ist darauf hinzuweisen, daß für die einzelnen Komponenten der erfindungsgemäßen Binde jeweils geeignete Materialien aus dem zur Verfügung stehenden Angebot nach den üblichen Kriterien für die Konstruktion solcher Hygieneartikel auszusuchen und geeignete Konstruktionsmaßnahmen zu treffen sind. Zu letzteren wird nur beispielhaft darauf hingewiesen, daß die Klebstoff-Streifen 117 so ausgelegt sein sollten, daß sie die Oberfläche der Trägerlage 107 nur im möglichst geringen Maße gegen einen Flüssigkeitsdurchtritt verschließen, so daß die saugaktive Oberfläche der Binde möglichst groß bleibt. Es kann also ein Sprühkleber oder in dünnen Fäden schmelzgeblasener Kleber verwendet werden. Auch ein Aufschweißen oder-siegeln durch Laser, Ultraschall oder Wärme ist denkbar.

In den Fig. 19 und 20 schließlich sind Ausführungsformen des erfindungsgemäß absorbierenden Artikels dargestellt, bei welchen der Saugkörper 205 eine plissierte Form aufweist. Die in den genannten Figuren gezeigten Artikel enthalten darüber hinaus auch eine plissierte Abdeckschicht 203, welche in Verbindungspunkten 202 mit einer äußeren Abdecklage 201 verbunden sind (Fig. 19). Die in Fig. 19 gezeigte Ausführungsform zeigt darüber hinaus eine flüssigkeitsundurchlässige Abdeckschicht 204, welche im Fachjargon auch als "Polybaffle" bezeichnet wird.

Der Saugkörper, welcher mindestens eine gewellte Bahn enthält, kann neben den vorstehend erwähnten Materialien Kardenvlies und Spinnvlies, beispielsweise auch eine luftgelegte Faserbahn, Laminate oder das weiter unten näher spezifizierte Material Coform enthalten. Zellstoff-Kunstfaser-Mischungen können problemlos bis zu 25 g/g Flüssigkeit aufnehmen. Zusätzlich zu einer saugfähigen Bahn kann der Saugkörper auch noch zusätzlich einen Superabsorber enthalten. Diese Superabsorber haben eine Flüssigkeitsaufnahmefähigkeit von etwa 1 bis 500 g/g physiologischer Kochsalzlösung. Für körpereigene Flüssigkeit beträgt das Aufnahmevermögen etwa 1 g/g bis 80 g/g.

Als flüssigkeitsdurchlässige Abdeckmaterialien sind insbesondere mehrlagige Laminate aus gelochten Folien und Spinnvliesen bzw. mehrlagige Laminate aus gelochten Folien und Kardenvliesen mit Flächengewichten von etwa 20 bis 200 g/m², vorzugsweise 30 bis 100 g/m², insbesondere 42 g/m², und/oder Dicken von 0,15 bis 4,0 mm, vorzugsweise 0,3 bis 1,5 mm, insbesondere 0,50 mm, geeignet.

Als Bestandteil des Saugkörpers eignen sich Materialien aus Kardenvlies, Spinnvlies, Bahnmaterial aus Zellstoff/Kunstfasermischung (Coform), eine verfestigte luftgelegte Zellstoffbahn (airlaid material), oder eine luftgelegte Zellstoff/Kunstfasermischung-Bahn (thermo fixiertes airlaid material). Weiterhin geeignet sind Prism- und Coform-Materialien, saugfähige Bahnen aus luftgelegtem Zellstoff, Zellstoff/Kunstfasermischungen oder Kunstfaservliese mit einem Flächengewicht von etwa 12 bis etwa 400 g/m², vorzugsweise 50 bis 150 g/m², insbesondere 100 g/m² und/oder Dicken von 0,1 bis 3 mm, vorzugsweise 0,2 bis 2 mm, insbesondere 1 mm.

Die körperseitige Abdecklage sollte vorzugsweise hydrophile Eigenschaften aufweisen. Mögliche weitere Transportschichten und/oder Materialien zwischen körperseitiger Abdecklage und Saugkörper können sowohl hydrophil als auch hydrophob sein.

Es versteht sich für den Fachmann von selbst, daß die plissierte Abdecklage 203 auch durch eine nichtplissierte Schicht ersetzt werden kann. Auch wenn nur zumindest ein Teil des Saugkörpers plissiert ist, werden bei einem erfindungsgemäßen Hygieneartikel die seitliche Auslaufsicherheit erhöht und eine bessere Längsverteilung der aufgenommenen Flüssigkeit erreicht. Des weiteren ist selbstverständlich, daß sowohl die flüssigkeitsdurchlässige Abdecklage als auch die flüssigkeitsundurchlässige Abdecklage bei einem absorbierenden Artikel mit einem zumindest teilweise gewellten Saugkörper aus den vorstehend näher beschriebenen Materialien aufgebaut sein können.

In Fig. 21 ist eine Ausführungsform des erfindungsgemäß absorbierenden Artikels dargestellt, bei welchem der Saugkörper 205 eine plissierte Form mit unterschiedlicher Höhe der Wellen aufweist. Die Wellenhöhe H₁ im Zentrum des absorbierenden Artikels beträgt dabei vorzugsweise maximal den zehnfachen Wert der Wellenhöhe H₂ in Randbereichen des Artikels.

Die hergestellten längsgewellten Bahnen (plissierten Bahnen) wurden auf ultradünne Saugkörper aufgebracht. In Tabelle 2 sind die Leistungsdaten entsprechender absorbierender Artikel zusammengefaßt.

Die in Tabelle 2 spezifizierten Materialien Prism, Ekotec und Coform weisen im einzelnen die folgenden Eigenschaften auf:
- Prism:: Spezial-Spinnvlies mit hohem Volumen, einseitig oder zweiseitig behandelt; Flächengewichte von etwa 12 bis etwa 80 g/m². Prism 0,5 entspricht einem Flächengewicht von etwa 17 g/m². Prism 1,0 entspricht einem Flächengewicht von etwa 35 g/m².
- Ekotec:: high loft Kardenvlies, welches mittels heißer Luft getrocknet ist (hot through air dried), hohes Volumen; Flächengewichte von etwa 12 bis etwa 70 g/m².
- Coform:: Zellstoff/Polypropylenmischung, Zellstoff zerfasert mit gesponnenen PP-Fasern vergefestigt; Flächengewichte etwa 40 bis etwa 400 g/m².

Die einseitige oder zweiseitige Behandlung des Prism-Materials mittels eines Arivage-Verfahrens bewirkt eine bessere Flüssigkeitsaufnahme und ein schnelleres Eindringen der Flüssigkeit, speziell wenn das Material hydrophiliert wird.

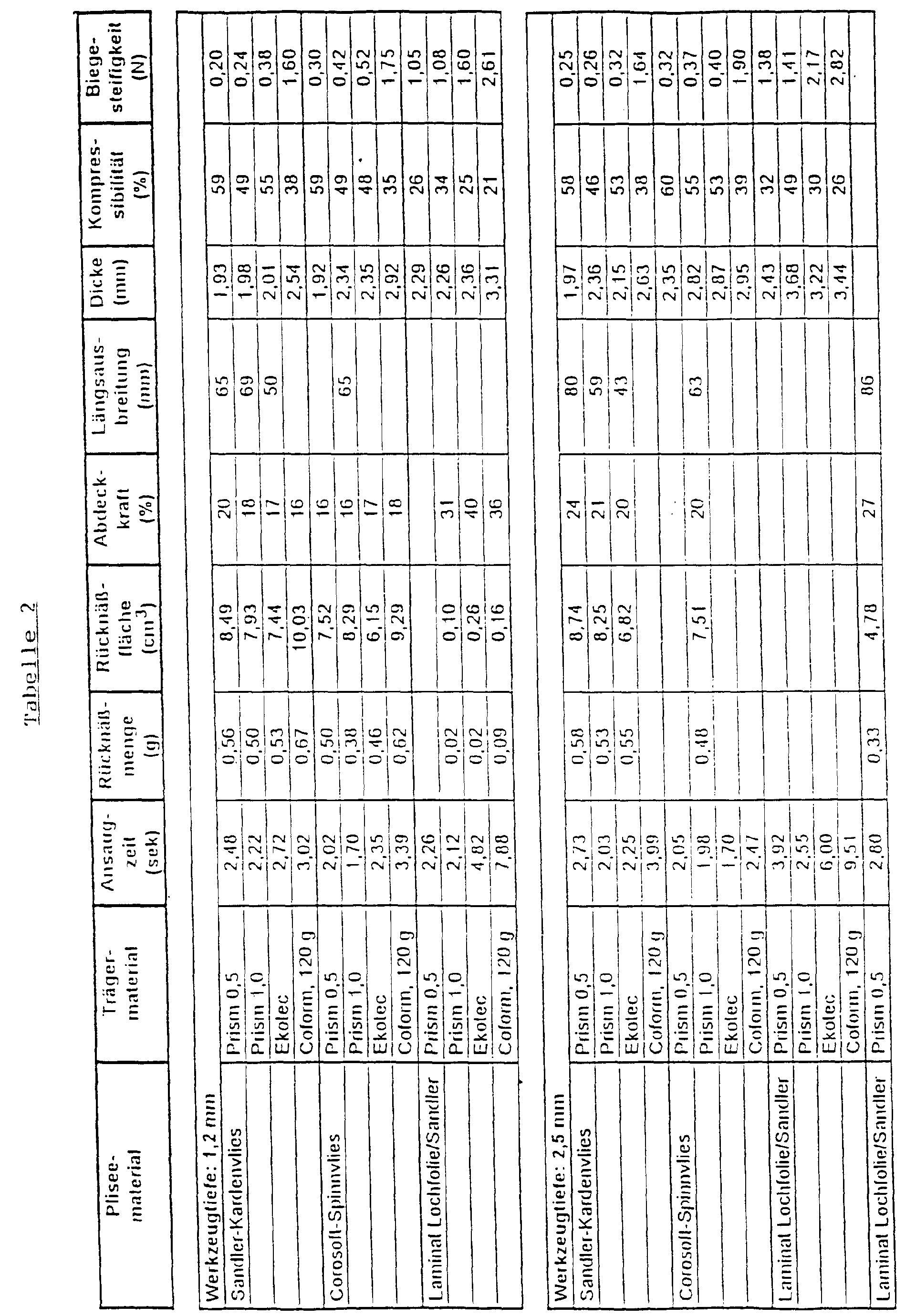

## Patentansprüche

1. Absorbierender Artikel mit
- einer flüssigkeitsundurchlässigen Abdecklage,
- einer flüssigkeitsdurchlässigen Abdecklage, und
- einem zwischen flüssigkeitsundurchlässiger Abdecklage und flüssigkeitsdurchlässiger Abdecklage angeordneten Saugkörper,
wobei die flüssigkeitsundurchlässige Abdecklage und/oder der Saugkörper zumindest in Teilbereichen eine gewellte Form aufweisen und der Saugkörper mindestens eine gewellte Bahn enthält,
wobei der Saugkörper ein Kardenvlies, ein Spinnvlies, ein Bahnmaterial aus einer Zellstoff-Kunstfaser-mischung, eine verfestigte luftgelegte Zellstoffbahn, eine luftgelegte Zellstoff-Kunstfasermischung oder Kunstfaservlies enthält, und
wobei das Kardenvlies und/oder das Spinnvlies ein Flächengewicht von 6 bis 80 g/m², vorzugsweise 10 bis 30 g/m², insbesondere 16 bis 18 g/m², und/oder eine Dicke von 0,05 bis 1,5 mm, vorzugsweise 0,1 bis 0,5 mm, insbesondere 0,13 bis 0,18 mm, und das Bahnmaterial aus einer Zellstoff-Kunstfasermischung, die verfestigte luftgelegte Zellstoffbahn, die luftgelegte Zellstoff-Kunstfasermischung und/oder das Kunstfaservlies ein Flächengewicht von 12 bis 400 g/m², vorzugsweise 50 bis 150 g/m², insbesondere 100 g/m², und/oder eine Dicke von 0,1 bis 3 mm, vorzugsweise 0,2 bis 2 mm, insbesondere 1 mm, aufweisen.

2. Absorbierender Artikel nach Anspruch 1, wobei das Kardenvlies oder das Spinnvlies hydrophile Eigenschaften aufweisen.

3. Absorbierender Artikel nach einem der Ansprüche 1 oder 2, wobei der Saugkörper zusätzlich zu der gewellten Bahn absorbierendes Material enthält.

4. Absorbierender Artikel nach Anspruch 3, wobei das zusätzliche absorbierende Material eine Zellstoff-Kunstfaser-Mischung und/oder ein Superabsorber ist.

5. Absorbierender Artikel nach Anspruch 4, wobei die Zellstoff-Kunstfaser-Mischung eine Flüssigkeitsaufnahmefähigkeit von 1 g/g bis 25 g/g und der Superabsorber eine Flüssigkeitsaufnahmefähigkeit von 1 g/g bis 500 g/g aufweist.

6. Absorbierender Artikel nach einem der Ansprüche 1 bis 5, wobei die flüssigkeitsdurchlässige Abdecklage ein Material enthält, wobei das Spinnvlies ein Flächengewicht zwischen 6 und 80 g/m², vorzugsweise 10 bis 30 g/m², insbesondere 16 g/m², und/oder eine Dicke von 0,05 bis 1,5 mm, vorzugsweise 0,1 bis 0,5 mm, besonders bevorzugt 0,1 bis 0,2 mm, insbesondere 0,13 mm, aufweist und das Kardenvlies ein Flächengewicht zwischen 6 und 80 g/m², vorzugsweise 10 bis 30 g/m², insbesondere 18 g/m² und/oder eine Dicke von 0,05 bis 1,5 mm, vorzugsweise 0,1 bis 0,5 mm, besonders bevorzugt 0,1 bis 0,2 mm, insbesondere 0,18 mm, aufweist und die mehrlagigen Laminate ein Flächengewicht von 20 bis 200 g/m², vorzugsweise 30 bis 100 g/m², insbesondere 42 g/m² und/oder eine Dicke von 0,15 bis 4,0 mm, vorzugsweise 0,3 bis 1,5 mm, insbesondere 0,5 mm aufweisen.

7. Absorbierender Artikel nach einem der Ansprüche 1 bis 6, wobei dieser eine Damenbinde, Windel oder Inkontinenzeinlage ist.
